# EUROPEAN PATENT APPLICATION

(11) **EP 1 938 805 A1**
(43) Date of publication of application: **02.07.2008**
(21) Application number: 06127072.4
(22) Date of filing: 22.12.2006
(51) Int. Cl.: A61K 9/20, A61K 9/28, A61K 31/437

(54) **Monolithic sustained release zolpidem tablets**

(71) Applicant: LEK Pharmaceuticals d.d., 1526 Ljubljana (SI)
(72) Inventor: Kuhar, Polonca, 1000 Ljubljana (SI); Opara, Jerneja, 1000 Ljubljana (SI)
(74) Representative: Kröger, Bernd

(57) **Abstract**

The invention provides a sustained release pharmaceutical tablet with substantially uniform release of zolpidem or a salt thereof, and release of more than 90% zolpidem in less than 2 hours.

## Description

### FIELD OF THE INVENTION

The present invention belongs to the field of pharmaceutical technology and relates to a pharmaceutical formulation with modified release properties.

More particularly, the invention relates to a pharmaceutical formulation of matrix tablets comprising an active substance, typically zolpidem, which is released with continuous and uniform dissolution kinetics.

### BACKGROUND OF THE INVENTION

Zolpidem tartrate is an imidazopyridine which is reported to have similar sedative properties to the benzodiazepines, but minimal anxiolytic, muscle relaxant and anticonvulsive properties. Zolpidem appears to act by binding to the benzodiazepine receptor component of the GABA receptor complex. It has a rapid onset and short duration of action and is used as a hypnotic in the short term management of insomnia since it decreases time to sleep onset and increases duration of sleep with little apparent effect on sleep phases. Zolpidem is rapidly absorbed from gastrointestinal tract, peak plasma concentrations being reached within 3 hours.

US Patent 6,514,531 describes controlled release dosage forms comprising zolpidem or a salt thereof. The dosage forms are adapted to release zolpidem over a predetermined time period, according to a biphasic profile of dissolution, where the first phase is an immediate release phase and the second phase is a prolonged release phase. Embodiments intended to avoid abuse are also provided. The examples of the US Patent provide coated tablets, filled capsules or multi-layer tablets. As illustrated by Figure 6, the biphasic profile of dissolution results in a step-wise release profile, with an initial burst in the first phase, and the remainder in the later second phase. The described biphasic profile is achieved by manufacturing tablets with two separate layers which consist of different ingredients and need to be compressed on a special tableting machine with two filling stations. Since two separate granulates have to be prepared and the amount of each granulate in every tablet has to be carefully adjusted and constant through the entire tableting process, such technology is complicated and time consuming. The robustness of the process can also be at risk due to problems with weight and content uniformity.

WO 01/00181 relates to timed dual release dosage forms comprising a short acting hypnotic or a salt thereof. The dissolution profile comprises two release pulses, the first being an immediate release usually lasting up to 30 minutes, and the second being delayed by a fixed time usually between 50 and 200 minutes. The examples relate to coated pellets used to fill capsules or compressed to give tablets. As illustrated by Figure 1, the timed dual release dosage form results in a step-wise release profile, with an initial burst in the first pulse, and the remainder in the later second pulse.

### SUMMARY OF THE INVENTION

In a first aspect, the invention concerns a pharmaceutical formulation which can be prepared by a simple and cost effective process. The formulation comprises a tablet core from which a therapeutic dose of an active substance which is highly soluble in water according to the Biopharmaceutics Classification, BCS Class 1, can be released in a sustained release manner.

In another aspect, the invention concerns a monolithic sustained release dosage form comprising zolpidem or salts thereof adapted to release the drug over a predetermined time period, with substantially uniform dissolution kinetics. Such a formulation has no need for layers, granules, beads, coated subunits or any other form of release modifying microenvironment. It can consist of a uniform tablet core, prepared from a dry powder mixture by direct compression and optionally coated.

More particularly, a sustained release pharmaceutical tablet of this invention can give substantially uniform release of zolpidem or a salt thereof for a period of at least 60 minutes. There can be release of more than 90% zolpidem in less than 110 minutes.

A tablet of this invention can comprise a core that includes a matrix of sustained release material, and zolpidem or a salt thereof adhered to a glidant. Such a tablet can give sustained release of the zolpidem or salt thereof as a single phase, that is a monophasic release.

In a related aspect, the invention concerns a pharmaceutical formulation comprising zolpidem or salts thereof and at least one excipient from each of the following groups: hydrophilic swelling polymer of suitable viscosity, filler, glidant and lubricant, and optionally acidifying agent. In general, a mention of zolpidem includes the salts and especially the hemitartrate.

In a further aspect, the invention concerns a pharmaceutical formulation described above which can be further coated with a protective coating, which has no appreciable impact on release of active substance from the final dosage form.

In another aspect, the invention concerns a process for the preparation of such pharmaceutical formulations comprising preparation of a blend of the ingredients for the tablet core, compression of such mixture followed by pan-coating of prepared tablet cores to obtain film coated tablets.

In an additional aspect, the invention concerns a use of such pharmaceutical formulations with zolpidem or pharmaceutically acceptable salts thereof where many polymorphic forms of active ingredient exist, since it enables preservation of the original polymorph form throughout the manufacturing process.

Furthermore, the present invention provides a method of inducing sedation in a person which involves administering or taking a tablet of the present invention.

### DETAILED DESCRIPTION OF THE INVENTION

The present invention provides a pharmaceutical dosage form, more specifically tablets, which can be prepared by compression of a dry powder mixture. Since addition of any solvent during this manufacturing step is not required, such a formulation is of particular advantage in the case of active substances which are prone to solvent-triggered degradation or transition of polymorph form.

Dry powder mixtures can present a special challenge for formulators due to their flow properties, compression behaviour and their tendency to segregate. Therefore, the composition of the formulation, particle size of ingredients and manufacturing procedure (such as mixing, sequence of excipient addition) has to be carefully evaluated and optimised during formulation development. Therefore, to assure content uniformity, active substance is adhered to a glidant such as colloidal silicon dioxide, and the filler or combination of fillers, which are the main constituents of the formulation, are selected to provide good powder flow and aid compressibility of the formulation.

The new dosage form according to the present invention with its sustained release enables specific drug release which results in rapid initial absorption from the gastrointestinal tract sufficient for onset of sleep followed by maintenance of sufficient blood concentration to enable maintenance of sleep.

The new dosage form was designed to obtain a rapid initial absorption from the gastrointestinal tract and extended plasma concentrations in comparison with an immediate release dosage form. The rapid initial absorption enables the rapid onset of action and sleep induction. Due to the modified release properties of the formulation the prolonged action and consequently sleep maintenance is achieved. The described pharmacokinetic properties of the new formulation are achieved with the suitable *in vitro* dissolution profile in 0.01 M HCl at 37°C, when measured using the method described in the US pharmacopoeia with the rotating paddle apparatus at a paddle speed of 50rpm.

The dissolution of a formulation is modified and sustained in a such extent that typically 45% of a drug is released in 30 minutes and 60% of the drug is released in 45 minutes. This portion of the drug is mainly responsible for the peak plasma concentrations and enables the rapid onset of action. The remaining drug is released afterwards and enables the extended plasma concentrations that are necessary for the prolonged action. Typically more than 70% of drug is released in 60 minutes and more than 90% of drug is released in 110 minutes. The release of a drug is sustained and uniform over the whole dissolution profile, with more than 90% zolpidem released at 2 hours. Thus, in less than 2 hours the drug release is almost completed to allow the drug elimination and condition of alertness on awakening.

The sustained release drug product of the present invention can be characterised by a specific *in vitro* profile of dissolution when measured in a rotating paddle apparatus in 0.01 M HCl at 37ºC using the method described in the US pharmacopoeia with the rotating paddle apparatus at a paddle speed of 50rpm. The profile of embodiments, including preferred embodiments, can be described by the percentage release at four time points:

| time | typical amount released | preferred amount released | more preferred amount released |
|---|---|---|---|
| t₁=30 min | 40% to 50% | 42% to 48% | about 45% |
| t₂=45 min | 50% to 70% | 55% to 65% | about 60% |
| t₃=60 min | more than 75% | more than 70% | more than 70% |
| t₄=110 min | more than 90% | more than 90% | more than 90% |

It is preferred that the release is substantially uniform, with less than 20% deviation from a straight line drawn through the values for the amount released at two time points separated by 60 minutes. More preferably there is less than 15% deviation, more preferably less than 10%. The two time points are typically 15 and 75 minutes. More generally the second time point is ordinarily less than 90 minutes.

Release of active substance from the dosage form described in present invention can be precisely regulated by changing the ratio between matrix forming polymer and soluble filler in the formulation.

We surprisingly found out that the *in vitro* release profile and its robustness changes if the sequence of addition of excipients is changed. It can be explained in that it is the content uniformity of the matrix forming polymer and its homogeneity throughout the tablet volume which determines the homogeneity of the gel layer formed in presence of water or other dissolution media, and therefore dictates the dissolution rate. Consequently, the manufacturing process was optimised to achieve best possible homogeneity and minimise relative standard deviation in dissolution profiles between tablets.

In the following description, the percentages for the ingredients of the tablets are by weight of the tablet core, before application of any coating, unless the context clearly requires otherwise.

The active substance is usually present in an amount of less than 60%, preferably less than 40%, more preferably less than 20%. For zolpidem, the amount is usually less than 10%, more preferably less than 7%, and typically about 5%. In particular, the tablets of this invention typically contain from 4 to 16 mg of zolpidem, calculated as the base, more preferably about 6.25 mg or about 12.5 mg.

In addition to the active substance, tablet cores can comprise the following excipients: matrix forming polymer, filler, glidant, lubricant and optionally acidifying agent.

The filler can be chosen among powdered cellulose, sorbitol, mannitol, various types of lactose, phosphates and the like. Most commonly, lactose can be used as a filler. It may be of any commercially marketed form, either in anhydrous or monohydrate form. Preferably, spray dried lactose in used due to its excellent flow properties. The amount of the filler can be between 60 and 85%, preferably more than 65% and most preferably more than 70%.

Matrix forming polymer can be chosen among hydrophilic or hydrophobic polymers such as derivatives of cellulose (for example methylcellulose, hydroxypropyl cellulose, hypromellose, ethylcellulose); polyvinylpirolidone (for example povidone, crospovidone, copovidone); polymethacrylates (for example Eudragit RS, RL); lypophillic components (for example glyceryl monostearate, glyceryl behenate); and various other substances such as for example hydroxypropyl starch, polyethylene oxide and carrageenan. Most commonly, hydrophilic swelling polymers of suitable viscosity such as hypromellose are used, preferably in amounts above 5%, more preferably above 8% and most preferably between 10 and 13%. The preferred viscosity of hydrophilic polymer is between 60 and 140 cP, more preferably between 80 and 120 cP and most preferably around 100 cP, if measured in the form of 2% aqueous solution at 20°C.

Glidant is preferably selected among one of the following excipients: colloidal silicon dioxide, talc, magnesium stearate, calcium stearate, aluminium stearate, palmitic acid, stearic acid, stearol, cetanol and polyethylene glycol. Preferably, colloidal silicon dioxide is used. The core contains 0 to 5% of colloidal silicon dioxide.

Lubricant can be selected among one of the following excipients: stearic acid, magnesium stearate, calcium stearate, aluminium stearate, sodium stearyl fumarate, talc, hydrogenated castor oil and polyethylene glycols.

Tablet cores can be prepared by simple and cost effective manufacturing procedures conventional in pharmaceutical technology. Preferably, tablets are prepared by direct compression of dry powder mixture or granulate, obtained by dry granulation. A dry powder mixture is prepared by the following procedure: zolpidem or its salts, preferably zolpidem hemitartrate is mixed and adhered onto colloidal silicon dioxide or other glidant with high specific surface area. Separately, hypromellose or other matrix-forming polymer is mixed with the filler, usually the same amount of lactose, to obtain a homogenous mixture, which is then added to the adhered active substance followed by addition of any remaining excipients usually except for the lubricant. Finally, magnesium stearate or other lubricant is admixed and the dry powder mixture is compressed into tablets.

A suitable protective film coating for light protection and taste masking comprises a hydrophilic polymer usually selected among cellulose derivatives, such as methylcellulose, hydroxypropyl cellulose, hypromellose, ethylcellulose, hydroxyethyl cellulose or combinations thereof. Preferably, a mixture of hydroxypropyl cellulose and hypromellose is applied. The coating is typically less than 5% by weight of the coated tablet, typically 3 to 4%. Such a coating has no significant influence on the dissolution behaviour of the coated tablets.

### EXAMPLES

The present invention is illustrated but in no way limited by the following examples:

### Example 1

| | amount per tablet (mg) |
|---|---|
| **Core** | |
| Zolpidem hemitartrate | 12,50 |
| Hypromellose 2208 | 27,00 |
| Lactose, spray dried | 184,18 |
| Colloidal sillicon dioxide | 1,92 |
| Magnesium stearate | 2,40 |
| Potassium bitartrate | 12,00 |

### Manufacturing procedure:

### Tablet cores:

Zolpidem hemitartrate is mixed and adhered onto colloidal silicon dioxide. Separately, hypromellose and lactose are homogenised and added to adhered active substance. Finally, magnesium stearate is admixed, then dry powder mixture is compressed into tablet cores.

Dissolution profile of tablet cores, described in example 1 (USP ap.2, 50rpm, 900 mL, 0.01 M HCl):

| t (min) | % dissolved |
|---|---|
| 15 | 30.1 |
| 30 | 46.8 |
| 45 | 59.2 |
| 60 | 72.5 |
| 75 | 82.4 |
| 90 | 90.7 |
| 105 | 96.2 |
| 120 | 98.7 |

### Example 2

| | amount per tablet (mg) |
|---|---|
| **Core** | |
| Zolpidem hemitartrate | 12,50 |
| Hypromellose 2208 | 24,00 |
| Lactose, spray dried | 199,18 |
| Colloidal silicon dioxide | 1,92 |
| Magnesium stearate | 2,40 |

| **Coating** | |
|---|---|
| Hypromellose 2910 | 3,84 |
| Hydroxypropylcellulose | 1,28 |
| Polyethylene glycol 400 | 0,80 |
| Titanium dioxide | 1,68 |
| Talc | 0,40 |
| Water | 68,18 |

### Manufacturing procedure:

### Tablet cores:

Zolpidem hemitartrate is mixed and adhered onto colloidal silicon dioxide. Separately, hypromellose and lactose are homogenised and added to adhered active substance. Finally, magnesium stearate is admixed, then the dry powder mixture is compressed into tablet cores.

### Coating:

Hypromellose, hydroxypropylcellulose, polyethylene glycol, titanium dioxide and talc are dispersed in demineralised water. Tablet cores are pan-coated with the described dispersion.

### Example 3

| | amount per tablet (mg) |
|---|---|
| **Core** | |
| Zolpidem hemitartrate | 12,50 |
| Hypromellose 2208 | 27,00 |
| Lactose, spray dried | 196,18 |
| Colloidal silicon dioxide | 1,92 |
| Magnesium stearate | 2,40 |

| **Coating** | |
|---|---|
| Hypromellose 2910 | 2,56 |
| Hydroxypropylcellulose | 2,56 |
| Polyethylene glycol 400 | 0,80 |
| Ferric oxide red | 0,02 |
| Titanium dioxide | 1,66 |
| Talc | 0,40 |
| Water | 68,18 |

The method of preparation may be analogous as with Example 2.

Dissolution profile of tablets, described in example 3 (USP ap.2, 50rpm, 900 mL, 0.01 M HCl):

| t (min) | % dissolved |
|---|---|
| 15 | 22.3 |
| 30 | 41.8 |
| 45 | 58.4 |
| 60 | 73.5 |
| 75 | 88.5 |
| 90 | 95.4 |
| 105 | 96.7 |
| 120 | 96.9 |

### Example 4

| | amount per tablet (mg) |
|---|---|
| **Core** | |
| Zolpidem hemitartrate | 12,50 |
| Hypromellose 2208 | 27,00 |
| Lactose, spray dried | 172,18 |
| Colloidal silicon dioxide | 1,92 |
| Potassium bitartrate | 24,00 |
| Magnesium stearate | 2,40 |

| **Coating** | |
|---|---|
| Hypromellose 2910 | 2,56 |
| Hydroxypropylcellulose | 2,56 |
| Polyethylene glycol 400 | 0,80 |
| Titanium dioxide | 1,68 |
| Talc | 0,40 |
| Water | 68,18 |

The method of preparation may be analogous as with Example 2.

## Claims

1. A sustained release pharmaceutical tablet with substantially uniform release of zolpidem or a salt thereof for a period of at least 60 minutes, and release of more than 90% zolpidem in less than 110 minutes.

2. A tablet according to claim 1, where release of zolpidem is in accordance with the following:
| time | amount released |
|---|---|
| t₁=30 min | 40% to 50% |
| t₂=45 min | 50% do 70% |
| t₃=60 min | more than 75% |
| t₄=110 min | more than 90% |

3. A tablet according to claim 2, where release of zolpidem is in accordance with the following:
| time | amount released |
|---|---|
| t₁=30 min | 42% to 48% |
| t₂=45 min | 55% TO 65% |
| t₃=60 min | more than 70% |
| t₄=110 min | more than 90% |

4. A tablet according to claim 3, where release of zolpidem is in accordance with the following:
| time | amount released |
|---|---|
| t₁=30 min | about 45% |
| t₂=45 min | about 60% |
| t₃=60 min | more than 70% |
| t₄=110 min | more than 90% |

5. A tablet according to any preceding claim, which contains from 4 to 16 mg of zolpidem, calculated as base.

6. A tablet according to any preceding claim, wherein the zolpidem is present as zolpidem hemitartrate.

7. A tablet according to any preceding claim which comprises zolpidem or a salt thereof, matrix-forming polymer, filler, glidant, and lubricant.

8. A tablet according to claim 7, which further comprises acidifying agent.

9. A tablet according to any preceding claim which comprises zolpidem or a salt thereof, hypromellose, spray-dried lactose, colloidal silicon dioxide and magnesium stearate, potassium bitartrate and a protective coating.

10. A process for preparing a sustained release pharmaceutical composition of zolpidem which process comprises forming a dry blend of active ingredient and glidant, admixing with other ingredients including sustained release excipient, and forming tablets.
